# EUROPÄISCHE PATENTSCHRIFT

(11) **EP 1 165 122 B1**
(45) Veröffentlichungstag und Bekanntmachung des Hinweises auf die Patenterteilung: **11.09.2002**
(21) Anmeldenummer: 00918815.2
(22) Anmeldetag: 20.03.2000
(51) Int. Cl.: A61K 38/58, A61P 7/02

(54) **VERWENDUNG VON MOLEKULARGEWICHTSERWEITERTEM HIRUDIN ALS ANTIKOAGULANS BEI DER EXTRAKORPORALEN NIERENERSATZTHERAPIE**
USE OF MOLECULAR WEIGHT-AMPLIFIED HIRUDIN AS AN ANTICOAGULANT IN EXTRACORPOREAL RENAL REPLACEMENT THERAPY
HIRUDINE A MASSE MOLECULAIRE ETENDUE UTILISEE COMME ANTICOAGULANT DANS LA DIALYSE RENALE EXTRACORPORELLE

(30) Priorität: 08.04.1999 DE 19915862
(43) Veröffentlichungstag der Anmeldung: 02.01.2002
(73) Patentinhaber: Max-Planck-Gesellschaft zur Förderung der Wissenschaften e.V. Berlin, 80539 München (DE)
(72) Erfinder: NOWAK, Götz, D-99097 Erfurt (DE); BUCHA, Elke, D-99094 Erfurt (DE)
(74) Vertreter: Albrecht, Thomas, Dr.
(86) Internationale Anmeldenummer: EP0002446
(87) Internationale Veröffentlichungsnummer: WO00061121

(56) Entgegenhaltungen:
- EP-A- 0 345 616
- WO-A-95/15183
- DE-A- 19 715 504
- GB-A- 2 247 239
- US-A- 5 663 141
- ESSLINGER H U ET AL: "Pharmacodynamic and safety results of PEG-Hirudin in healthy volunteers." THROMBOSIS AND HAEMOSTASIS, (1997 MAY) 77 (5) 911-9. , XP000952170
- VAN WYK V ET AL: "The effect of r-hirudin vs. heparin on blood-membrane interactions during hemodialysis." CLINICAL NEPHROLOGY, (1997 DEC) 48 (6) 381-7. , XP000952174
- SCHMIDMAIER G ET AL: "A NEW BIODEGRADABLE POLYLACTIC ACID CORONARY STENT-COATING, RELEASING PEG-HIRUDIN AND A PROSTACYCLINE ANALOG, REDUCES BOTH PLATELET ACTIVATION AND PLASMATIC COAGULATION" JOURNAL OF THE AMERICAN COLLEGE OF CARDIOLOGY,XX,XX, Bd. 29, Nr. 2, SUPPL, 16. März 1997 (1997-03-16), Seite 354A XP002068341 ISSN: 0735-1097
- BUCHA E ET AL: "In vitro study of r-hirudin permeability through membranes of different haemodialysers." NEPHROLOGY, DIALYSIS, TRANSPLANTATION, (1999 DEC) 14 (12) 2922-6. , XP000952175

## Beschreibung

Die Erfindung betrifft die Verwendung von molekulargewichtserweitertem Hirudin ausgewählt aus Polyethylenglykol-gekoppeltem Hirudin, Polyzucker-gekoppeltem Hirudin, Fettsäure-gekoppeltem Hirudin, Dextranhirudin, Albuminhirudin, γ-Globulinhirudin und Transferrin-Hirudin zur Herstellung eines keine Autoimmunerkrankung induzierenden Antikoagulans für die extrakorporale Nierenersatztherapie. Insbesondere betrifft die Erfindung die Verwendung von molekulargewichtserweitertem Hirudin zur Herstellung eines Antikoagulans für die extrakorporale Mierenersatztherapie, das keine Thrombozytopenie Typ II (HIT II) induziert, wie dies bei den bisher verwendeten Heparinen der Fall ist.

Die extrakorporale Nierenersatztherapie stellt eine seit mehr als 30 Jahren eingeführte und sichere Methode dar, die bei chronischer Niereninsuffizienz bzw. Nierenlosigkeit die Eliminationsfunktion der Nieren durch spezielle Module, die im Gegenstromverfahren das Blut von Fremdstoffen reinigen, ersetzt. Die relativ großen Oberflächen der hierbei benutzten Kapillardialysätoren (0,5 bis 2 m²) und die dabei auftretenden Flüssigkeits- und Proteintransfers haben eine hohe thrombogene Potenz, so daß sich die Anwendung von gerinnungshemmenden Medikamenten während der Hämodialyse dringend erforderlich macht. In der klinischen Routine werden nahezu ausschließlich Heparin bzw. heparinanaloge Substanzen verwendet. Die Heparinapplikation bei nierenfunktionsgestörten oder nierenlosen Patienten hat eine ganze Reihe von Nebenwirkungen gezeigt (Osteoporose, veränderte Blutfettzusammensetzung u.a.m.), die aber von den Patienten toleriert werden mußten, da keine effizienten Alternativen zur Verfügung standen. In den letzten Jahren hat sich zusätzlich eine weitere schwere Nebenwirkung des Heparins herausgestellt, die in 0,5 bis 10% aller Heparin-behandelten Fälle mehr oder weniger klinisch relevant wird, die heparininduzierte Thrombozytopenie Typ II. Hierbei handelt es sich um eine iatrogene Zweitkrankheit mit schwerer thrombogener Tendenz.

Die Patienten entwickeln hierbei gegen das Heparin neutralisierende Prinzip, den Plättchenfaktor 4, im Komplex mit Heparin einen Antikörper. Dieser Antikörper erkennt nur die komplexierte Plättchenfaktor 4-Heparinstruktur. Gegen Heparin allein oder gegen den Plättchenfaktor 4 allein ist dieser Antikörper nicht reagibel bzw. kreuzreagibel. Umgekehrt zeigen alle heparinartigen Substanzen, die mit dem Plättchenfaktor 4 Komplexe bilden können, auch eine Kreuzreaktion. Dazu gehören neben den fraktionierten Heparinen auch polysulfatierte Medikamente vom Typ des Orgarans oder sulfatierte Polyzuckerstrukturen, die aus pflanzlichen Quellen gewonnen werden können. Diese Immunerkrankung verläuft bei den Hämodialysepatienten zunächst lange Zeit symptomlos, weil die aktivierten Blutplättchen mit dem Antikörper und dem durch die Injektion im Blut erschienenen Heparin und dem Plättchenfaktor 4 großmolekulare Aggregate bilden, die in den Kapillardialysatoren hängenbleiben. Am Ende der Dialysebehandlung ist der Patient noch nicht heparinfrei, so daß auch zwischen den Dialysesitzungen diese Komplexbildung vonstatten geht und dementsprechend in der Peripherie mehr oder weniger erkennbare oder krankheitserzeugende Veränderungen in der Mikrozirkulation von Organen oder Gefäßgebieten hervorruft. Dies ist vermutlich die wesentliche Ursache dafür, daß Hämodialysepatienten viel häufiger als andere Patienten an Herz-Kreislauf-Erkrankungen oder weiteren Organausfällen im Verlauf der Nierenersatztherapie erkranken bzw. daran sterben.

Ohne entsprechende Therapie liegt die Sterblichkeit nach dem Auftreten erster klinischer Symptome bei ca. 30%. Diese Nebenwirkung erfordert eine strikte Vermeidung weiterer Applikationen von Heparin. Als Ersatzantikoagulans wurde bisher ausschließlich Orgaran eingesetzt. Diese Substanz ist ein Mischprodukt aus Dermatansulfat, Heparansulfat und 5 bis 10% Heparin. Sehr viele Patienten sind gegen dieses Medikament primär kreuzreaktiv bzw. zeigen nach längerer Behandlung mit dieser Substanz ebenfalls allergene Reaktionen. Dieselbe Kreuzreaktion ist auch für low molecular weight-Heparine zu finden. Patienten ohne Nierenfunktionseinschränkung können seit Mai 1997 erfolgreich mit dem neuen direkten Antithrombin Hirudin behandelt werden.

Es hat nicht an Versuchen gefehlt, das Hirudin auch für die Indikation der Nierenersatztherapie zu erschließen. Da jedoch Hirudin ausschließlich in unveränderter Form über die Nieren eliminiert wird, muß dementsprechend die Hirudindosierung beim nierenkranken Patienten mit größter Vorsicht eingestellt werden. Ein nicht vorhandenes drug monitoring (welches erst in den letzten 1 bis 2 Jahren in die klinische Praxis eingeführt wurde), ließ das Hirudin bisher für die Indikation Hämodialyse nicht praktisch relevant erscheinen. Eine wiederholte Applikation war erst möglich, nachdem eine lückenlose Kontrolle des Blutspiegels vor und nach der Dialyse zur Bestimmung der minimal wirksamen Hirudinblutspiegel zur Verfügung stand (Ecarin Clotting Time, US-Patent Nr. 5 547 850 vom 20.08.1996, PCT/EP93/00161). Erste Erfahrungen wurden bei ultimativer Hirudinantikoagulation bei Patienten mit dialysepflichtiger HIT II erhalten (Nowak, G., Bucha, E., Brauns, I., Czerwinski, R.: Anticoagulation with r-Hirudin in Regular Haemodialysis with Heparin-Induced Thrombocytopenia (HIT II), Wien Klin. Wochenschr. 109, Nr. 10, 1997), ebenso auch bei klinischen Studien nach einmaliger bzw. mehrmaliger Applikation von rHirudin.

rHirudin war bei präklinischen und ersten klinischen Untersuchungen für die Hämodialyse nur einsetzbar, wenn low flux-Polysulfon- oder low flux-Zellulose-Dialysatoren verwendet wurden, weil rHirudin diese Dialysemembranen nicht passiert. Alle anderen low flux-Dialysatorenmaterialien wie auch alle high flux-Materialien werden von rHirudin relativ schnell passiert, so daß eine universelle Verwendung als Antikoagulans in der täglichen Hämodialysepraxis erschwert wird, da unter diesen Bedingungen eine routinemäßige Blutspiegel-adaptierte Dosierungseinstellung nicht möglich ist (vgl. Bucha, E., Kreml, R., Nowak, G.: In Vitro Study of Transmembrane Hirudin Passage Using Different Types of Dialyzers, Poster, 23, Ccngress of the European Renal Association, Amsterdam 1996; Bucha, E., Nowak, G., Butti, A.: Clinical Studies of Blood-Level Controlled Repeated Application of rHirudin in Haemodialysis Patients, Thromb. Haemost., Supplement June 1997; und Nowak, G., Bucha, E.: Quantitative Determination of Hirudin in Blood and Body Fluids, Sem. Thromb. Hemost. 22, Nr. 2, 1996).

Eine Verwendung von Hirudin bei speziellen high flux-Dialysatoren, die sich in Anwendung bei Intensivtherapiepatienten befinden, ist ebenfalls stark erschwert, weil diese sogenannten Hämofiltrations- oder Hämodiafiltrationssysteme extrem große Poren haben, durch die Toxine und Peptide bis zu einer molekularen Masse von 45 kDa penetrieren. rHirudin passiert diese Membran ebenfalls wie die konventionellen high flux-Membranen und ist innerhalb kurzer Zeit auf der Dialysatseite zu finden. Bei der Verwendung von rHirudin müßten den Patienten extrem hohe Mengen infundiert werden. Eine Steuerung dieser Hirudintherapie ist schlecht möglich und zu teuer, so daß eine routinemäßige Anwendung für diese Indikation nicht möglich ist.

Es besteht somit ein Bedarf nach einem Antikoagulans, das zur Gerinnungshemmung während der extrakorporalen Nierenersatztherapie (Hämodialyse) unabhängig von den genutzten Hämodialysatoren bzw. Hämofiltern verwendet werden kann und das gut verträglich ist und insbesondere weder Autoimmunerkrankungen hervorruft noch eine Kreuzreaktivität zu während einer bestehenden Immunreaktion gebildeten Antikörpern aufweist. Bei der erfindungsgemäßen Verwendung soll insbesondere keine Thrombozytopenie Typ II hervorgerufen werden und keine Kreuzreaktivität zu Antikörpern gegen Plättchenfaktor 4-Heparin-Komplex bestehen. Die Gerinnungshemmung soll einfach, kostengünstig und unter gut meßbaren Bedingungen durchgeführt werden können. Das Antikoagulans soll ferner die Membranen von Hämodialysatoren bzw. Hämofiltern nicht passieren können.

Überraschenderweise wurde gefunden, daß im Gegensatz zu rHirudin molekulargewichtserweitertes Hirudin Kapillardialysatoren vom low und high flux-Typ nicht passiert, vom Patienten gut vertragen wird und keine Autoimmunerkrankungen, insbesondere keine Thrombozytopenie, hervorruft und nicht mit entsprechenden Antikörpern kreuzreagiert. Molekulargewichtserweitertes Hirudin eignet sich somit sehr gut als Antikoagulans für die Hämodialyse.

Die Erfindung betrifft somit die Verwendung von molekulargewichtserweitertem Hirudin ausgewählt aus Polyethylenglykol-gekoppeltem Hirudin, Polyzucker-gekoppeltem Hirudin, Fettsäuregekoppeltem Hirudin, Dextranhirudin, Albuminhirudin, γ-Globulinhirudin und Transferrin-Hirudin zur Herstellung eines keine Autoimmunerkrankung induzierenden Antikoagulans für die extrakorporale Nierenersatztherapie. Insbesondere betrifft die Erfindung die Verwendung von molekulargewichtserweitertem Hirudin wie vorstehend definiert zur Herstellung eines keine Thrombozytopenie induzierenden und nicht mit Autoimmunantikörpern kreuzreagierenden Antikoagulans für die extrakorporale Nierenersatztherapie.

Das molekulargewichtserweiterte Hirudin ist ausgewählt aus Polyethylenglykol-gekoppeltem Hirudin, Protein-gekoppeltem Hirudin, DNA-RNA-gekoppeltes Hirudim, Polyzucker-gekoppeltem Hirudin, z.B. Dextranhirudin, Albuminhirudin, γ-Globulinhirudin, Transferrin-Hirudin, Fettsäure-gekoppeltem Hirudin (z.B. Palmitoylhirudin, Farnesylhirudin). Bevorzugt wird Polyethylenglykol-gekoppeltes Hirudin verwendet.

Die Molekulargewichtserweiterung des Hirudins erfolgt allgemein durch Substanzen, so daß die so molekulargewichtserweiterten Hirudine keine Bindungstendenz zu Proteinen, Blutplättchen und anderen Blutbestandteilen zeigen. Die Molekulargewichtserweiterung wird vom Fachmann in Abhängigkeit des zu therapierenden Zustands und des Verabreichungswegs gewählt. Sie liegt im allgemeinen im Bereich zwischen 500 und 500.000 Da, bevorzugt 1.000 bis 250.000 Da, weiter bevorzugt 3.000 bis 150.000 Da, noch bevorzugter 5.000 bis 25.000 Da. Die molekulargewichtserweiterten Hirudine werden nach an sich bekannter Weise unter Verwendung üblicher Formulierungshilfsstoffe entsprechend dem jeweiligen Verabreichungsweg konfektioniert.

Der für die Hämodialyse erforderliche Hirudin-Blutspiegel sollte vorzugsweise zwischen 0,4 und 1,0 µg/ml Plasma eingestellt sein. In diesem Blutspiegelbereich wird die Aktivierung des Gerinnungssystems auf den Polymeroberflächen der dabei verwendeten Dialysemodule effizient gehemmt. Der einzustellende Hirudin-Blutspiegel hängt jedoch von dem physiologischen Zustand des zu behandelnden Patienten ab und wird von einem Arzt in Abhängigkeit von Patienten-typischen Faktoren eingestellt.

Der Blutspiegel bei Verwendung von molekulargewichtserweitertem Hirudin ist vorzugsweise mittels intravenöser Bolüsinjektion von z.B. 0,01 bis 0,1 mg/kg Polyethylenglykol-Hirudin zu erreichen (für diskontinuierliche extrakorporale Nierenersatztherapie). Für die Verwendung im Bereich der Intensivtherapie zur Hämofiltration/Hämodiafiltration ist die Applikation von PEG-Hirudin auch über extravasale Applikation möglich, wie intramuskuläre, intraperitoneale, pulmonale oder subkutane Anwendung. Dazu sollte mindestens zwei Stunden vor Beginn der Hämofiltration die Gabe z.B. des Polyethylenglykol-Hirudins in einer Dosierung von 0,2 bis 1,5 mg/kg, bevorzugt 0,5 bis 0,7 mg/kg subkutan erfolgt sein. Weitere subkutane Injektionen würden erst nach jeweils 48 Stunden (0,1 bis 0,5 mg/kg, bevorzugt 0,2 bis 0,3 mg/kg PEG-Hirudin) erforderlich sein. Die Dosisangaben berechnen sich dabei nach dem aktiven Hirudin, unabhängig von der zur Molekulargewichtserweiterung verwendeten inerten Substanz. Bei Protein-gekoppelten Hirudinen (z.B. Transferrin, Albumin o.a. körpereigene, blutpflichtige Proteine) und PEG-Hirudinen mit einem Molekulargewicht >50 kDa hat sich das folgende Dosierungsschema bewährt: Erste Hirudingabe: 0,05 mg/kg als Bolus, bei jeder weiteren Injektion 0,01 mg/kg als Bolus direkt vor Beginn der Dialyse. Bei kontinuierlichen Verfahren (Hämodialyse oder Hämofiltration) reicht die Erstapplikation von 0,05 mg/kg, nach 48 h 0,01 mg/kg als Nachinjektion jeweils jeden zweiten Tag während des Wechsels des Hämofilters als einmalige intravasale Applikation. Parenterale Injektionsverfahren (s.c., i.m.) eignen sich für diese Sondergruppe der molekulargewichtserweiterten Hirudine aufgrund ihrer Molekülgröße nicht. Alle molekulargewichtserweiterten Hirudine von 500 Da bis 500 kDa können dem Patienten direkt intravasal (i.v. oder i.a.) appliziert werden. Bis zu einer Molekülgröße von etwa 50 kDa kann die Applikation auch extravasal, parenteral erfolgen, also intramuskulär, subkutan, intrakutan, intrapulmonal. Über diese Grenzgröße hinaus ist das nicht möglich, weil dann diese großmolekularen Substanzen nicht mehr aus den extravasalen Injektionsdepots in das Gefäßinnere aufgenommen werden können. Die Zellzwischenräume sind für diese Molekülgröße unpassierbar. Wenn nun bewußt sehr große Moleküle als Hirudinträger verwendet werden, speziell Proteine oder Nukleinsäuremakromoleküle und andere entsprechende Moleküle, die über 50 kDa groß sind, kann davon ausgegangen werden, daß diese Moleküle nicht mehr die Gefäßzirkulation verlassen, d.h. sie verteilen sich nur im Blutraum des Körpers. Das Verhältnis zwischen Blutraum und extrazellulärem Flüssigkeitsraum, welche die beiden Verteilungsgrößen für Moleküle unter 50 kDa darstellen, ist etwa 1:5, d.h. es muß bei den sehr großen Hirudinmolekülen eine bedeutend geringere Menge intravenös oder intraarteriell appliziert werden, damit sich keine zu hohen Blutspiegel entwickeln.

Die genaue Dosierung hängt von dem verwendeten molekulargewichtserweiterten Hirudin, dem Zustand des Patienten, der Art der Verabreichung und der Behandlungsdauer ab und wird vom Arzt individuell ermittelt. Ebenso wird die Häufigkeit der Verabreichung vom Arzt festgelegt. Die Kontrolle der therapeutischen Blutspiegel kann problemlos mit der Ecarin Clotting Time als therapeutisches drug monitoring erfolgen (Method of Determining Hirudin, US-Patent Nr. 5 547 850, PCT/EP93/00161). Bei Beachtung der pharmakokinetischen Daten von PEG-Hirudin (Esslinger, H.U. et al.: Pharmacodynamic and Safety Results of PEG-Hirudin in Healthy Volunteers, Thromb. Haemost. 77 (5), 1997) ist eine Überdosierung der PEG-Hirudinsubstanz ausgeschlossen. Bei eventueller iatrogener Überdosierung ist durch die Verwendung eines Polymethylmethacrylat-Dialysators der PEG-Hirudinspiegel sehr schnell in therapeutische Bereiche absenkbar (PMMA-Membranen mit Polyethylenglykol-gekoppelten Wirksubstanzen, DPA 197 15 504.9 vom 14.04.1997). Damit steht für die PEG-Hirudin-Antikoagulation bei Nierenersatzverfahren ein "Antidot" zur Verfügung, da PMMA-Dialysatoren Handelsprodukte der Fa. Toray, Japan, sind.

Die erfindungsgemäßen molekulargewichtserweiterten Hirudine werden in an sich bekannter Weise entsprechend dem Verabreichungsweg formuliert. Dabei werden übliche Formulierungshilfsmittel, Excipientien und Korregenzien verwendet. Bevorzugt werden Substanzen zugesetzt, die die Löslichkeit und Stabilität von getrockneten Präparaten verbessern, wie beispielsweise Zuckerverbindungen (Mannit, Dextran), inerte Proteine (Albumin) oder Kollagene (Prionex). Zusätzlich können zur Verbesserung der Lagerung Salze, vor allem Puffersalze, wie z.B. Bicarbonate oder Hydrogenphosphate und andere auf dem Gebiet der Formulierung von Puffersystemen übliche Salzverbindungen zugesetzt werden-. Bevorzugt werden Ampullen hergestellt, die derart sind, daß gebrauchsfertige Injektionslösungen entstehen, die die gewichtsbezogene Dosierung berücksichten, z.B. 1-, 2- oder 5 mg-Ampullen. Es ist zweckmäßig, die zur Lösungsverbesserung bzw. als Formulierungshilfsmittel zugesetzten Substanzen maximal im molekularen Verhältnis von 1:1 zuzusetzen. Als günstig erwiesen sich auch Zusätze im Bereich zwischen ein Fünftel und 1:1, bezogen auf das Molekulargewicht der molekulargewichtserweiterten Hirudinsubstanz. Die Lösung sollte so formuliert werden, daß das gebrauchsfertige molekulargewichtserweiterte Hirudin eine blutisotone Konzentration aufweist. Es ist daher bei der Formulierung darauf zu achten, daß Zusätze von Puffersalzen oder Kochsalz im Bereich einer möglichst optimalen Blutisotonie erfolgen, um die Verträglichkeit der Präparate bei Applikation in die Blutbahn zu gewährleisten. Das molekulargewichtserweiterte Hirudin kann auch in gefriergetrockneter Form in luftdichten Ampullen oder Injektionsfläschchen bereitgestellt werden, um dann vor der Anwendung mit beispielsweise physiologischer Kochsalzlösung pharmazeutischer Qualität auf die Gebrauchsstärke verdünnt zu werden.

Die erfindungsgemäße Verwendung eignet sich für alle handelsüblichen Hämodialysatoren.

Die Erfindung wird anhand der nachstehenden Beispiele näher erläutert:

### Beispiel 1

### Applikationsschema für PEG-Hirudin (10-kDa-PEG-Hirudin) zur Antikoagulation bei diskontinuierlichen Hämodialyseverfahren mit Verwendung aller high-flux- oder low-flux-Dialysatoren (mit Ausnahme von PMMA-Dialysatoren)

- Bei der ersten Hämodialyse wird intravenös eine Bolusinjektion von 0,08 mg/kg 10-kDa-PEG-Hirudin direkt vor der Hämodialysebehandlung vorgenommen.
- Bei den weiteren Hämodialysebehandlungen wird eine i.v. Bolusinjektion von 0,06 mg/kg 10 kDa-PEG-Hirudin vorgenommen.
- Die Kontrolle des PEG-Hirudinspiegels erfolgt mit der Ecarin Clotting Time am Ende der Hämodialysebehandlung. Der therapeutische Blutspiegelbereich beträgt 0,5 bis 0,8 µg/ml.

### Beispiel 2

### I.v. Applikationsschema für PEG-Hirudin (10-kDa-PEG-Hirudin) bei kontinuierlichen Dialyseverfahren (CVVHD, CAVHD)

- Direkt vor Beginn der kontinuierlichen Blutwäsche erfolgt lokal vor dem verwendeten Dialysator eine i.v. Applikation von 0,1 mg/kg 10-kDa-PEG-Hirudin.
- 24 Stunden bzw. 48 Stunden später werden die PEG-Hirudin-Blutspiegel mittels Ecarin Clotting Time kontrolliert.
- Nach 24 Stunden wird ein optimaler Blutspiegelbereich von 0,8 bis 0,5 µg/ml, nach 48 Stunden von 0,4 bis 0,6 µg/ml erhalten.
- Nach Dialysatorwechsel erfolgt eine Nachapplikation von 0,02 mg/kg 10-kDa-PEG-Hirudin.

### Beispiel 3

### S.c. Applikationsschema für PEG-Hirudin (10-kDa-PEG-Hirudin) bei kontinuierlichen Dialyseverfahren

(a) Bei Patienten mit noch vorhandener Nierenfunktion:
   - 2 Stunden vor Beginn der Hämodialyseverfahren erfolgt eine subkutane Applikation von 0,25 bis 1,0 mg/kg, bevorzugt 0,6 mg/kg PEG-Hirudin.
   - 24 Stunden und 48 Stunden nach Beginn der Behandlung wird der Blutspiegel kontrolliert.
   - Alle 2 bis 4 Tage erfolgt eine Nachinjektion von 0,1 bis 1 mg/kg, bevorzugt 0,3 mg/kg PEG-Hirudin.
(b) Bei Patienten mit fehlender Nierenfunktion:
   - Zu Beginn der Behandlung erfolgt eine s.c. Applikation von 0,2 bis 1,0 mg/kg, bevorzugt 0,4 mg/kg.
   - 24 Stunden und 48 Stunden nach Behandlungsbeginn erfolgt eine p.i. Blutspiegelkontrolle.
   - Alle 4 Tage werden 0,01 bis 0,25 mg/kg, bevorzugt 0,1 mg/kg PEG-Hirudin nachinjiziert.

### Beispiel 4

### Applikation von PEG-Hirudin beim Zustand nach chronischer Glomerulonephritis

Patient: W.B., 58 Jahre, männlich. Zustand nach chronischer Glomerulonephritis, dialysepflichtig seit 4,7 Jahren, Patient wurde mit 6900 IE Heparin antikoaguliert. Während der Hämodialyse gab der Patient zunehmende Beschwerden im Sinne von Durchblutungsstörungen in den Extremitäten an, Kältegefühl, Kribbeln, Parästhesien, die häufig erst mehrere Stunden nach der Dialysebehandlung bzw. in den Nacht- und Morgenstunden auftraten. Der Patient klagte während der Dialysen und auch in den dialysefreien Intervallen über Blutdruckprobleme und teilweise leichte Verwirrtheitszustände. In der HIT II-spezifischen Diagnostik (HIPA-Test) war ein positives Ergebnis erhalten worden. Die Zahl der Blutplättchen vor und nach der Hämodialyse lag im Durchschnitt zwischen 350 und 400/nl.

Der Patient wurde nach folgendem Dosierungsschema auf PEG-Hirudin eingestellt: 1. Hämodialyse: 0,1 mg/kg als Bolus, zweite bis 10. Hämodialyse: 0,05 mg/kg PEG-Hirudin, 11. Hämodialyse und weiter: 0,025 mg/kg. Mit Hilfe der Ecarin Clotting Time ist ein problemloses Monitoring während der Behandlung möglich. Der Blutspiegel von PEG-Hirudin schwankte zwischen 0,4 µg/ml vor Beginn der Hämodialyse und 1,0 µg/ml nach der Hämodialyse. Füllungsvolumina der Dialysatoren Typ GFS plus 11 (GAMBRO): unter Heparinapplikation-Mittelwert der drei letzten Heparin-antikoagulierten Dialysen: 87 ml, Füllungsvolumen während der PEG-Hirudin-Behandlung:HD 5-HD 6: 96 ml. Das Füllungsvolumen dieses Dialysatortyps wird mit 100 ml angegeben.

Der Patient litt unter einer arteriellen Durchblutungsstörung der Extremitäten. Die schmerzfreie Wegstrecke während der Heparin-antikoagulierten Hämodialyse betrug 150 bis 200 m, die schmerzfreie Wegstrecke unter PEG-Hirudin-Behandlung verlängerte sich, bei Kontrolle nach der 10. Hämodialyse war sie mehr als 700 m, und in der weiteren Behandlung gab der Patient eine weitere Verbesserung der schmerzfreien Wegstrecke an. Auch 8 Wochen nach Start der PEG-Hirudin-Behandlung wegen einer HIT II waren im Serum des Patienten HIT II-spezifische Antikörper nachweisbar.

Bereits nach den ersten PEG-Hirudin-Dialysen verminderten sich die parästhetischen Beschwerden und die nächtlichen Schmerzattacken. Nach der 7. PEG-Hirudin-Hämodialyse qab der Patient Beschwerdefreiheit während der Hämodialyse und in den dialysefreien Intervallen an.

Bewertung: PEG-Hirudin ist als Antikoagulans für die Hämodialyse bei HIT II-positiven Patienten geeignet. Die Symptome einer chronischen Mikrozirkulationsstörung bei permanent bestehender HIT II-Reaktion während der Heparinbehandlung des Patienten im Rahmen der Hämodialyse traten unter PEG-Hirudin nicht auf. Die aufgrund einer Claudicatio intermittens auftretende kurze schmerzfreie Wegstrecke verlängerte sich zusehends, und die Kreislaufdysregulation sowie das stark verminderte Füllungsvolumen der Dialysatoren waren nicht mehr vorhanden.

### Beispiel 5

### Applikation von PEG-Hirudin bei diabetischer Nephropathie

Patient: G.W., männlich, 57 Jahre, 54 kg. Der Patient wird wegen einer diabetischen Nephropathie seit 2,4 Jahren hämodialysiert. Der Patient hatte ein Harnrestvolumen von 1,2 bis 1,7 1. Die Creatininclearance war kleiner als 12 ml/min. Als Antikoagulans während der Hämodialyse wurde unfraktioniertes Heparin verwendet. Zusätzlich wurden die Dialysatoren (H 120, Braun-Melsungen) vor Beginn der Hämodialyse mit 500 Einheiten Heparin "geprimt". Der Patient erhielt 3000 Einheiten Heparin als Bolus und 3000 Einheiten Heparin als Infusion während der 3,5-stündigen Hämodialyse. Mit Beginn der Hämodialyse zeigte der Patient einen unverhältnismäßig hohen Blutdruckabfall um 35 mmHg vom Ausgangswert (135/85 mmHg). Der Patient klagte über Kribbeln in den Extremitäten, Unruhegefühl und teilweise Verwirrtheitszustände. Zwischen den Dialysesitzungen klagte der Patient über Kopfschmerzen, Unwohlsein und Appetitlosigkeit. Er gab an, daß seine Umgebung sich darüber beklage, daß er gereizt sei und einen gestreßten Eindruck mache. Die Prüfung auf HIT-positive Antikörper fiel bei diesem Patienten stark positiv aus. Sowohl der HIPA-Test als auch der ELISA waren positiv in allen geprüften Untersuchungsreihen.

Daraufhin wurde der Patient auf PEG-Hirudin umgestellt. Er erhielt als erste PEG-Hirudindosis 0,1 mg/kg, in den weiteren Hämodialysen wurde er bis zur Hämodialyse 5 mit 0,06 mg/kg behandelt, von der 6. bis auf weiteres wurde er mit 0,03 mg/kg weiterbehandelt. Die Füllungsvolumina am Ende der Hämodialyse waren bei Heparinbehandlung reduziert, die letzten zwei Hämodialysen mit Heparin hatten Füllungsvolumina von 71 und 73 ml. Bereits nach der 4. Hämodialyse mit PEG-Hirudin war ein Füllungsvolumen von 94 ml erreicht. Die Luftfallen und die Blutschlauchsysteme waren frei von zusätzlichen Koagula.

Der subjektive Zustand des Patienten hatte sich gebessert, sowohl das psychoorganische Syndrom als auch seine sonst angegebenen Beschwerden wie Kopfschmerzen, Übelkeit und Appetitlosikeit waren nicht mehr vorhanden. Der Patient zeigte noch einen geringen Blutdruckabfall mit Beginn der Hämodialyse, der im normalen Bereich lag. Die objektiven und subjektiven Befunde, die auf einen HIT II-Zustand hinwiesen, waren nach Umstellung der Antikoagulation auf PEG-Hirudin nicht mehr vorhanden. Die Blutplättchenzahlen, die bei dem Patienten während der letzten Heparindialysen stets Werte über 400/nl zeigten, waren während der PEG-Hirudin-Hämodialysen in den Bereich von 320 bis 350/nl abgefallen, wobei ein Trend zur weiteren Normalisierung deutlich nachweisbar war. Die Post-Dialysewerte für Leukozyten waren nahezu unverändert.

### Beispiel 6

### Applikation von PEG-Hirudin bei akutem Nierenversagen

Patient: L.A., weiblich, 69 Jahre, Zustand nach akutem Nierenversagen nach chronischer Pyelonephritis und interstitieller Nephritis. Die Patientin wurde seit 5 Monaten mit zunächst unfraktioniertem Heparin, dann mit fraktioniertem Heparin (Enoxaparin) dialysiert. Enoxaparin wurde in einer Dosis von 20 mg unmittelbar vor Beginn der Dialyse appliziert. Nach nicht korrekter Dialyse (Druckanstieg vor dem Dialysator) über die Dialysezeit von 4,5 h wurden zusätzlich 10 mg Enoxaparin über eine Dauerinfusionspumpe vor den Dialysator (F 50 S, Fresenius) appliziert. Die Patientin gab schwere Atemnotanfälle, Kreislauf- und Blutdruckabfall bis auf basale Werte von 80/30 mmHg kurz nach Beginn der Hämodialyse an. Die Patientin zeigte auch während der Dialyse eine massive Plättchenverminderung bis zu Werten von 100/nl. Der Nachweis von HIT II-Antikörpern mittels HIPA-Test und Plättchenfaktor 4-ELISA war positiv.

Die Patientin wurde dann auf PEG-Hirudin umgestellt. Restharnvolumen: 400 bis 600 ml/24 h, erste PEG-Hirudindosis: 0,1 mg/kg, zweite bis 10. Hämodialyse: 0,05 mg/kg PEG-Hirudin, 11. und weitere Hämodialysen: 0,02 mg/kg. Mit Beginn der PEG-Hirudinapplikation waren die Kreislaufveränderungen sowie die schwere akute pulmonale Insuffizienzreaktion der Patientin nicht mehr aufgetreten, die Blutplättchenkonzentration fiel während der Hämodialyse nicht ab. Die durch die bei der Patientin vorhandene relative Thrombozytopenie verminderten Plättchenzahlen (130 Plättchen/nl) stiegen innerhalb der ersten 6 Hämodialysen mit PEG-Hirudin auf über 200/nl an. Das seit mehreren Wochen bestehende Juckreiz- und Hautreaktionssyndrom hatte sich innerhalb von wenigen Hämodialysen zurückgebildet, die Patientin war beschwerdefrei und wies 6 Monate nach Beginn der PEG-Hirudin-Dialyse keinen HIT II-Antikörper mehr im Blut auf. Der mittels drug-monitoring-Methode eingestellte PEG-Hirudin-Blutspiegel lag bei der Patientin im Bereich von 0,5 µg/ml am Ende der Hämodialyse und war in den dialysefreien Intervallen von 1 bis 2 Tagen auf minimal 0,15 µg/ml abgefallen.

Zusammenfassend läßt sich feststellen, daß der bei allen drei Patienten nachweisbare basale PEG-Hirudin-Blutspiegel während der dialysefreien Intervalle im Bereich von 0,1 bis 0,4 µg/ml im subtherapeutischen Bereich liegt und völlig nebenwirkungsfrei ist. Bei keinem Patienten konnte auch während der Dialysebehandlung mit zwei- bis dreifach höheren Blutspiegeln eine Verlängerung der Blutungszeit nachgewiesen werden. Die permanente Antikoagulation verbessert das Offenhalten der arteriovenösen Shunts und ist für das bei allen Patienten vorhandene thrombophile Gesamtrisiko positiv zu bewerten.

Durch die Gabe des molekulargewichtserweiterten Hirudins wird verhindert, daß die so behandelten Patienten typische Autoimmunreaktionen zeigen. Hierzu gehören Blutdruckabfall, Atemnot, Schweißausbrüche, teilweise Hautreaktionen, wie Urtikaria oder umschriebene Arzneimittelexantheme, die als Sofortreaktion des auslösenden Agens, d.h. Heparin, beim Patienten sofort nachweisbar sind. Diese Autoimmunreaktionen können zu schwersten vital bedrohenden Zuständen führen. Typisch sind Reaktionen wie nicht mehr meßbarer Blutdruck, schwerste thrombogene Reaktionen mit Verlegung des Kapillardialysators, so daß eine effiziente Dialyse nicht weitergeführt werden kann. Die Dialysatoren müssen dann gewechselt werden (teilweise bis zu dreimal während einer Dialysesitzung), um die entsprechenden Filterfunktionen aufrechtzuerhalten. Im Gegensatz dazu treten diese Nebenwirkungen bei erfindungsgemäß mit molekulargewichtserweitertem Hirudin behandelten Patienten nicht mehr auf. In diesem Fall liegen stets stabile Kreislaufverhältnisse vor, und die Patienten leiden nicht unter subjektiven Wahrnehmungen, wie Hautkribbeln, flash-Reaktionen (Rötungen der Kopf- und Halsregion), Blutdruckabfall mit schweren Atemnotzeichen und Todesangst. Die Anwendung von molekulargewichtserweitertem Hirudin gemäß der Erfindung ist vor allem bei denjenigen Patienten indiziert, bei denen die vorstehend beschriebenen schwerwiegenden Symptome durch Heparin-Behandlung auftreten. Häufiger ist jedoch ein Zeichen für ein passageres HIT-II-Syndrom bei diesen Patienten, daß die Blutplättchenzahlen während der Dialyse stärker abfallen, und daß bei diesen Patienten vor Beginn der Dialyse entsprechende Antikörper nachgewiesen werden können, die aber nach der Hämodialyse nicht mehr aufgefunden werden, da sie dabei "verbraucht" wurden.

## Patentansprüche

1. Verwendung von molekulargewichtserweitertem Hirudin, ausgewählt aus Polyethylenglykol-gekoppeltem Hirudin, Polyzucker-gekoppeltem Hirudin, Fettsäure-gekoppeltem Hirudin, Dextranhirudin, Albuminhirudin, γ-Globulinhirudin und Transferrin-Hirudin zur Herstellung eines keine Autoimmunerkrankung induzierenden Antikoagulans für die extrakorporale Nierenersatztherapie.

2. Verwendung nach Anspruch 1, **dadurch gekennzeichnet, daß** die Autoimmunerkrankung eine heparininduzierte Thrombozytopenie Typ II ist.

3. Verwendung nach einem der Ansprüche 1 oder 2, **dadurch gekennzeichnet, daß** das molekulargewichtserweiterte Hirudin vasal oder extravasal zu verabreichen ist.

4. Verwendung nach einem der Ansprüche 1 bis 3, **dadurch gekennzeichnet, daß** das molekulargewichtserweiterte Hirudin in einer Dosierung zu verabreichen ist die zu einem Blutplasmaspiegel an Hirudin im Bereich von 0,4 bis 1,0 µg/ml Plasma führt.

## Claims

1. Use of molecular weight-amplified hirudin, selected from polyethylene glycol-coupled hirudin, polysugar-coupled hirudin, fatty acid-coupled hirudin, dextran-hirudin, albumin-hirudin, γ-globulin-hirudin and transferrin-hirudin, for the preparation of an anticoagulant in extracorporeal renal replacement therapy which does not induce autoimmune disease.

2. Use according to Claim 1, **characterized in that** the autoimmune disease is a type II heparin-induced thrombocytopenia.

3. Use according to Claim 1 or 2, **characterized in that** the molecular weight-amplified hirudin is to be administered vasally or extravasally.

4. Use according to one of Claims 1 to 3, **characterized in that** the molecular weight-amplified hirudin is to be administered in a dose which results in a blood plasma hirudin level ranging from 0.4 to 1.0 µg/ml.

## Revendications

1. Utilisation d'une hirudine dont la masse moléculaire a été augmentée, choisie dans le groupe formé par l'hirudine couplée à du polyéthylèneglycol, l'hirudine couplée à des polysaccharides, l'hirudine couplée à des acides gras, l'hirudine-dextrane, l'hirudine-albumine, l'hirudine-γ-globuline et l'hirudine-transferrine, pour la fabrication d'un agent anticoagulant n'induisant pas de maladies auto-immunes, pour la thérapie de remplacement rénal.

2. Utilisation selon la revendication 1, **caractérisée par le fait que** la maladie auto-immune est une thrombocytopénie de type II, induite par l'héparine.

3. Utilisation selon l'une des revendications 1 ou 2, **caractérisée par le fait que** l'hirudine dont on a augmenté la masse moléculaire, doit être administrée par voie vasculaire ou extravasculaire.

4. Utilisation selon l'une des revendications 1 à 3, **caractérisée par le fait que** l'hirudine dont on a augmenté la masse moléculaire, doit être administrée selon une posologie qui aboutit à un taux d'hirudine dans le plasma sanguin compris dans l'intervalle allant de 0,4 à 1,0 µg/ml de plasma.
